# EUROPEAN PATENT SPECIFICATION

(11) **EP 2 431 034 B1**
(45) Date of publication and mention of the grant of the patent: **10.08.2016**
(21) Application number: 10767158.8
(22) Date of filing: 23.04.2010
(51) Int. Cl.: A61K 31/4045, A61K 9/70, A61K 47/30, A61K 47/32, A61K 47/34, A61P 25/16

(54) **ADHESIVE PATCH-CONTAINING PACKAGE BAG AND METHOD FOR STORING ADHESIVE PATCH**
PACKUNGSBEUTEL MIT HEFTPFLASTERN UND VERFAHREN ZUR LAGERUNG DER HEFTPFLASTER
SAC DE CONDITIONNEMENT CONTENANT UN TIMBRE ADHÉSIF ET PROCÉDÉ DE STOCKAGE DU TIMBRE ADHÉSIF

(30) Priority: 24.04.2009 JP 2009106802
(43) Date of publication of application: 21.03.2012
(73) Proprietor: Hisamitsu Pharmaceutical Co., Inc., Tosu-shi, Saga 841-0017 (JP)
(72) Inventor: UCHIDA Naoyuki, Tsukuba-shi Ibaraki 305-0856 (JP); TAKAGI Yuka, Tsukuba-shi Ibaraki 305-0856 (JP); TAKADA Yasunori, Tsukuba-shi Ibaraki 305-0856 (JP)
(74) Representative: Grünecker Patent- und Rechtsanwälte PartG mbB
(86) International application number: PCT/JP2010/057245
(87) International publication number: WO 2010/123103

(56) References cited:
- WO-A1-97/11696
- WO-A2-2008/005240
- JP-A- 9 299 444
- JP-A- 11 188 075
- JP-A- 62 258 318
- JP-T- 11 506 462
- JP-T- 2001 518 058
- JP-T- 2002 509 879
- JP-U- H0 490 340
- US-A1- 2007 128 262
- Anonymous: "Neupro, INN-Rotigotine", EMEA , 2006, pages 1-40, XP002707237, Retrieved from the Internet: URL:http://www.ema.europa.eu/docs/en_GB/do cument_library/EPAR_-_Scientific_Discussio n/human/000626/WC500026394.pdf
- AYDOGMUS ET AL.: 'Highly sensitive and selective spectrophotometric and spectrofluorimetric methods for the determination of ropinirole hydrochloride in tablets, Spectrochimica acta.' MOLECULAR AND BIOMOLECULAR SPECTROSCOPY vol. 70, 2008, pages 69 - 78, XP022638014

## Description

### Technical Field

The present invention relates to a package bag containing a ropinirole-containing adhesive patch and a method for storing a ropinirole-containing adhesive patch.

### Background Art

Ropinirole was developed as a drug overcoming the limitations associated with L-Dopa therapy, and has been used for the treatment of Parkinson's disease. Further, research on ropinirole-containing transdermal preparations is attempted in consideration of avoidance of adverse reactions in the stomach and intestines and easiness of removal in the event of appearance of adverse reactions (see Patent Literatures 1 and 2).

Patent Literatures 1 and 2 propose various transdermal preparations, and one example thereof is an adhesive patch. An adhesive patch may be stored in a package bag so as to maintain its drug efficacy.

As such a package bag, for example, a package bag prepared by subjecting laminated sheets containing a backing layer, a barrier layer, a biaxially-drawn polypropylene film layer, and a sealant layer to three sided-sealing or four sided-sealing (see Patent Literature 3), a package bag made of laminated package materials in which a moisture absorption member layer is placed between a water permeable member layer and a blocking member layer (see Patent Literature 4), and a package bag in which at least a part of the inner surface is made of polyacrylonitrile (see Patent Literature 5) are known.
WO-A-97/11696 describes transdermal drug delivery, methods and drug delivery systems for administering ropinirole and other indolone derivatives transdermally, and pharmaceutical compositions formulated for transdermal administration.
"Neupro, INN-Rotigotine", EMEA, 2006, pages 1 to 40 describes transdermal formulations containing rotigotine.
US-A-2007/0128262 relates to a patch-containing pouch and to a method for inhibiting drug migration.

### Citation List

### Patent Literature

Patent Literature 1: JP-T-2001-518058
Patent Literature 2: JP-T-11-506462
Patent Literature 3: JP-A-2008-94426
Patent Literature 4: JP-A-2001-9985
Patent Literature 5: WO 2005/072675

### Summary of Invention

### Technical Problem

However, the present inventors have found that, when only the package bags of Patent Literatures 3 to 5 are used, problems of coloration of an adhesive patch and precipitation of crystals occur in an adhesive patch containing ropinirole and a pharmaceutically acceptable salt thereof. Further, the present inventors have found that the aforementioned coloration may be caused by the decomposition of a drug.

In view of the foregoing, an object of the present invention is to provide an adhesive patch-containing package bag that prevent the precipitation of crystals during storage and suppress the coloration, and further, inhibit the decomposition of ropinirole and a pharmaceutically acceptable salt thereof, and a method for storing an adhesive patch.

### Solution to Problem

In order to achieve the aforementioned object, the present invention provides an adhesive patch-containing package bag, comprising: a package bag; and an adhesive patch stored inside the package bag, wherein the package bag comprises a sealant layer as an innermost layer, an outer layer as an outmost layer and a barrier layer made of aluminum between the sealant layer and the outer layer, the package bag further comprises an oxygen scavenger as deoxygenation means, wherein the sealant layer is made of a resin selected from the group consisting of polyacrylonitrile, polyethylene and polyester, the adhesive patch comprises a backing, an adhesive layer laminated on at least one side of the backing and containing ropinirole and/or a pharmaceutically acceptable salt thereof, and a release liner covering the adhesive layer, and a relative humidity at 4°C is maintained at 35% or higher inside the package bag.

According to this adhesive patch-containing package bag, the precipitation of crystals during storage is prevented and the coloration is suppressed, and further, the decomposition of ropinirole is inhibited.

It is to be noted that a relative humidity at 4°C refers to the proportion (%) of the actual content of water vapor (kg·m⁻³) to the maximum amount of water vapor that can be held in a certain volume of air (kg·m⁻³: saturated humidity) at 4°C, with setting the maximum amount of water vapor at 100.

The aforementioned package bag have deoxygenation means. Owing to this, the preventive effects on the decomposition of ropinirole and the coloration of an adhesive patch with time are further improved.

The aforementioned package bag is formed of two or more layers, and an innermost layer of the package bag be is a sealant layer made of one resin selected from the group consisting of polyacrylonitrile, polyethylene, and polyester, and it is preferable that an outermost layer of the package bag be an outer layer made of one resin selected from the group consisting of polyethylene terephthalate, cellophane, and biaxially-drawn polypropylene.

The aforementioned package bag further include, between the aforementioned sealant layer and outer layer, a barrier layer made of aluminum.

It is preferable that the aforementioned adhesive layer contain at least one adhesive selected from the group consisting of an acrylic adhesive, a rubber adhesive, and a silicone adhesive.

Also, the present invention provides a method for storing an adhesive patch, comprising: storing an adhesive patch into a package bag, the adhesive patch comprising a backing, an adhesive layer laminated on at least one side of the backing and containing ropinirole and/or a pharmaceutically acceptable salt thereof, and a release liner covering the adhesive layer; and maintaining a relative humidity at 4°C inside the package bag at 35% or higher, wherein the package bag comprises a sealant layer as an innermost layer, an outer layer as an outmost layer and a barrier layer made of aluminum between the sealant layer and the outer layer, the package bag further comprises an oxygen scavenger as deoxygenation means, wherein the sealant layer is made of a resin selected from the group consisting of polyacrylonitrile, polyethylene and polyester.

According to this storing method, the precipitation of crystals during storage is prevented and the coloration is suppressed, and further, the decomposition of ropinirole is inhibited.

### Advantageous Effects of Invention

According to the adhesive patch-containing package bag and the method for storing an adhesive patch of the present invention, the precipitation of crystals of ropinirole or a pharmaceutically acceptable salt thereof contained in the adhesive patch can be suppressed, and further, the decomposition of a drug can be inhibited. For this, the drug content in an adhesive patch can be maintained by storing the adhesive patch in a package bag after production of the adhesive patch until before use. Accordingly, upon application, a sufficient amount of ropinirole can be absorbed across the skin.

### Brief Description of Drawings

[Figure 1] Figure 1 is a cross-sectional view illustrating a preferred embodiment of the adhesive patch-containing package bag of the present invention.
[Figure 2] Figure 2 is a graph showing the results of the measurement of a relative humidity inside the adhesive patch-containing package bags of Examples and Comparative Examples.

### Description of Embodiments

Hereinbelow, the embodiments of the present invention will be described in detail with reference to the drawings.

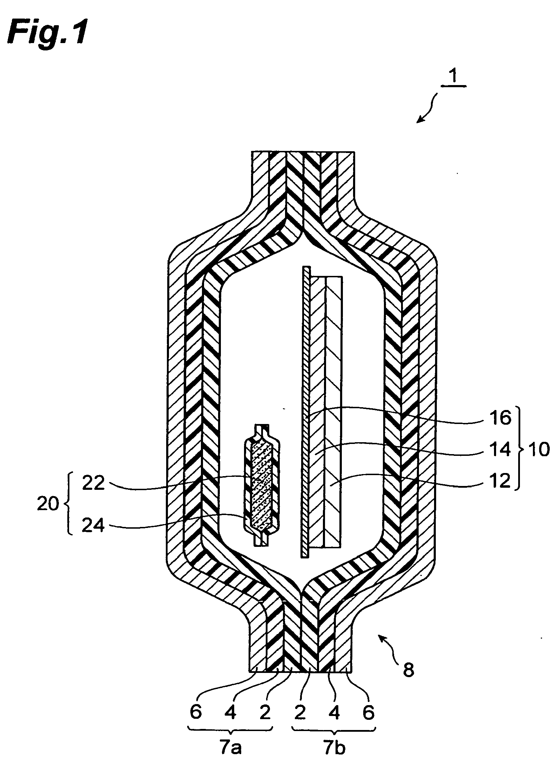

Figure 1 is a cross-sectional view illustrating a preferred embodiment of the adhesive patch-containing package bag of the present invention. An adhesive patch-containing package bag 1 contains a package bag 8 composed of a pair of laminated packing materials 7a and 7b placed opposite to each other and an adhesive patch 10 stored in the space inside the package bag 8. Also, in the adhesive patch-containing package bag 1 illustrated in Figure 1, a packaged oxygen scavenger 20 is further stored in the package bag 8 as deoxygenation means. In the adhesive patch-containing package bag 1 having the above configuration, a relative humidity at 4°C is maintained at 35% or higher in the package bag 8 as will be described later.

The package bag 8 packing the aforementioned adhesive patch 10 is composed of a pair of almost rectangular-shaped laminated packing materials 7a and 7b placed opposite to each other. The laminated packing materials 7a and 7b are rectangular film-like laminations having a configuration in which a sealant layer 2, a barrier layer 4, and an outer layer 6 are laminated in this order from the inside. Also, the laminated packing materials 7a and 7b placed opposite to each other are bonded together around the periphery thereof, whereby the package bag 8 is closed at the periphery all around. It is to be noted that peripheral bonding of these laminated packing materials 7 can be performed by heat sealing or using an adhesive agent.

Among each layer composing the laminated packing materials 7a and 7b, the material of the outer layer 6 is not particularly limited; however, examples thereof include a resin film selected from cellophane, paper, synthetic paper, biaxially-drawn polypropylene (OPP), polyester (such as polyethylene terephthalate), nylon, polyvinylidene chloride (PVDC), and polyvinyl alcohol (PVA), among which polyethylene terephthalate, cellophane, and biaxially-drawn polypropylene (OPP) are preferably used. Use of the aforementioned resin as the outer layer 6 enables physical protection of the inner layer of the bag and suppression of the occurrence of alteration such as corrosion in the outer surface of the package bag.

Also, the outer layer 6 may be composed of a plurality of layers each made of different materials, for example, it may be a layer in which a layer composed of cellophane and a layer composed of polyethylene are laminated.

The thickness of the outer layer 6 is formed within a range of preferably 5 µm to 600 µm, more preferably 5 µm to 500 µm. When the thickness of the outer layer 6 is less than 5 µm, there is a tendency that stiffness decreases, leading to reduced physical strength and impaired handling properties and protective properties. Also, when the thickness of the outer layer 6 becomes thicker than 600 µm, while stiffness increases and physical strength is also improved, there are tendencies that the yield of production decreases and the production cost increases.

The barrier layer 4 is laminated for securing of the non-permeation of substances from outside and prevention of volatilization of the product stored within, and for assurance of stability of the adhesive patch inside the package bag. Although the material of the barrier layer is not particularly limited, examples thereof include aluminum foils, ethylene vinyl alcohol copolymer (EVOH) films, and films made of plastic films on which a metal such as aluminum or ceramic such as silicon oxide is vapor deposited, and aluminum foils are preferably used. Use of aluminum foils as the barrier layer 4 enables complete blockade of light rays, water vapor, and oxygen.

The sealant layer 2 is a resin layer for formation of a package bag by bonding the laminated packing materials composing the package bag by heat sealing, and it is preferably a thermoplastic resin layer. The material composing the sealant layer 2 includes low density polyethylene (LDPE), linear low density polyethylene (LLDPE), polyester and polyacrylonitrile (PAN).

When the sealant layer 2 is composed of a material having high drug permeability, a phenomenon in which the interfaces of the layers of the laminated packing materials are detached during storage of the adhesive patch (i.e., delamination) may occur; however, the use of polyacrylonitrile, polyethylene, or polyester as the inner surface layer prevents permeation of the components of the adhesive patch, whereby the problem of delamination is overcome. Particularly, the use of polyacrylonitrile, polyethylene, or polyester for the entire inner surface layer enables efficient inhibition of the decomposition of a drug. That is, even when an adhesive patch containing a drug moves inside the package bag after being stored therein, the decomposition of the drug can be prevented.

The thickness of the sealant layer 2 may be thick enough to allow bonding of the laminated packing materials, for example, it may be approximately 10 µm to 50 µm.

The method for laminating each layer composing the aforementioned laminated packing materials 7a and 7b is not particularly limited, and extrusion lamination, dry lamination, hot melt lamination, and wet lamination are used.

The adhesive patch 10 contains a rectangular backing 12, an adhesive layer 14 laminated on almost the entirety of one side of the backing 12, and a release liner 16 covering the adhesive layer 14 and capable of being released from the adhesive layer 14.

Although the backing 12 is not particularly limited as long as it is a material capable of supporting the adhesive layer 14, it is desirably a material having excellent flexibility and capable of excellently maintaining the transferability of the drug, and either a stretchy or non-stretchy material is used, and a material capable of efficiently releasing the drug from the adhesive layer is preferable. Specifically, for example, a film, a sheet, or a lamination of these materials, a porous membrane, a foam, woven and non-woven cloth made of a synthetic resin such as polyethylene terephthalate, polyethylene, polypropylene, polybutadiene, ethylene/vinyl acetate copolymers, polyvinyl chloride, polyester, nylon, and polyurethane, or a paper material can be preferably used as the backing.

As the release liner 16, a resin film such as polyethylene terephthalate and polypropylene, and release-treated paper, can be used, and particularly, a film made of silicone-treated polyethylene terephthalate is preferably used.

The adhesive layer 14 is made of an adhesive composition containing an adhesive and a drug, namely, ropinirole or a pharmaceutically acceptable salt thereof (hereinbelow, these are collectively called a "ropinirole compound").

The content of the ropinirole compound in the adhesive composition is preferably 0.5 to 50 mass%, more preferably 1 to 30 mass% based on the total mass of the adhesive composition. When the drug content is less than 0.5 mass%, there is a tendency that an adequate drug efficacy cannot be obtained. Meanwhile, when the drug content exceeds 50 mass%, the adhesive layer cannot retain the drug, thus leading to a tendency to impair adhesive physical properties.

Although the adhesive in the adhesive composition is not particularly limited as long as it is an adhesive with the excellent adhesive and drug-releasing properties, one or two or more selected from natural rubber adhesives, synthetic rubber adhesives, acrylic adhesives, rubber adhesives, and silicone adhesives are preferably used. Among them, synthetic rubber adhesives and/or acrylic adhesives are preferred since they have the excellent adhesive and drug-releasing properties. Examples of the synthetic rubber adhesive include styrene block copolymers such as styrene-isoprene-styrene block copolymers (SIS), styrene-butadiene-styrene block copolymers (SBS), styrene-ethylene-butylene-styrene block copolymers (SEBS), and styrene-ethylene-propylene-styrene block copolymers (SEPS), and particularly, SIS is preferable.

The acrylic adhesive is not particularly limited as long as it is a copolymer containing at least acrylic acid or one (meth)acrylic ester represented by, 2-ethylhexyl acrylate, methyl acrylate, butyl acrylate, hydroxyethyl acrylate, or 2-ethylhexyl methacrylate. Specific examples of the acrylic adhesive include 2-ethylhexyl acrylate/vinyl acetate copolymers, 2-ethylhexyl acrylate/vinyl acetate/acrylic acid copolymers, 2-ethylhexyl acrylate/vinyl acetate/hydroxyethyl acrylate copolymers, 2-ethylhexyl acrylate/vinyl acetate/hydroxyethyl acrylate·acrylic acid copolymers, and 2-ethylhexyl acrylate/2-ethylhexyl methacrylate/dodecyl methacrylate copolymers, among which, particularly, 2-ethylhexyl acrylate/vinyl acetate copolymers and 2-ethylhexyl acrylate/vinyl acetate/acrylic acid copolymers are preferable.

Also, as the adhesive, an adhesive composition composed of a combination of plural kinds of the aforementioned adhesives is also preferable. Examples of such an adhesive composition include an adhesive composition composed of a combination of SIS and acrylic adhesives, and specifically, an adhesive composition in which SIS and 2-ethylhexyl acrylate/vinyl acetate/acrylic acid copolymers are combined is preferable.

The content of the adhesive in the aforementioned adhesive composition is preferably 10 to 95 mass% based on the total mass of the adhesive composition.

The aforementioned adhesive composition may contain tackifiers, and softeners, as needed.

Examples of the tackifier include cycloaliphatic saturated hydrocarbon resins, rosin derivatives (such as rosin, glycerin ester of rosin, hydrogenated rosin, glycerin ester of hydrogenated rosin, and rosin pentaerythritol ester), terpene resins, petroleum resins, or maleic acid resins. Among them, particularly, cycloaliphatic saturated hydrocarbon resins and glycerin ester of hydrogenated rosin are preferable. One of these tackifiers may be used alone or two or more thereof may be used in combination.

Examples of the softener include petroleum oil (such as paraffinic process oil, naphthenic process oil, and aromatic process oil), squalane, squalene, vegetable oils (such as almond oil, olive oil, camellia oil, castor oil, tall oil, and peanut oil), olefinic acid, silicone oil, dibasic esters (such as dibutyl phthalate and dioctyl phthalate), liquid rubber (such as polybutene and polyisoprene), liquid fatty acid esters (such as isopropyl myristate, hexyl laurate, diethyl sebacate, and isopropyl sebacate), diethylene glycol, polyethylene glycol, glycol salicylate, propylene glycol, dipropylene glycol, triacetin, triethyl citrate, or crotamiton. Among them, particularly, liquid paraffin, isopropyl myristate, and diethyl sebacate are preferable because they can impart an appropriate adherent property to the skin. One of these softeners may be used alone or two or more thereof may be used in combination.

It is to be noted that when including the tackifiers, and softeners in the adhesive composition, the content of each of these substances is preferably within the following range. That is, based on the total mass of the adhesive composition, the content of tackifier is preferably 10 to 90 mass%, and the content of softener is preferably 5 to 60 mass%.

Also, transdermal absorption promoters, dispersion aids, antioxidants, fillers, cross-linking agents, preservatives, and ultraviolet ray absorbers may be appropriately added to the aforementioned adhesive composition as needed.

Examples of the transdermal absorption promoter include aliphatic alcohols such as octyl dodecanol and isostearyl alcohol, fatty acids such as capric acid, fatty acid derivatives such as propylene glycol monolaurate, isopropyl palmitate, and isopropyl myristate, propylene glycol, polyethylene glycol, and lauric acid diethanolamide. One of these absorption promoters can be used alone, or two or more thereof can be used in combination.

In consideration of sufficient permeability of the active ingredient into the tissues, and local stimulation of a preparation, the blending amount of the absorption promoter is preferably 1 to 30 mass%, more preferably 3 to 20 mass%, particularly preferably 5 to 15 mass% based on the total mass of the adhesive composition.

Examples of the dispersion aid include inorganic minerals, polymer compounds, and organic acids. As the inorganic mineral, kaolin, talc, bentonite, zinc oxide, titanium oxide, zinc stearate, silicic acid compounds such as Aerosil and hydrous silica, magnesium aluminometasilicate, and dried aluminum hydroxide gel are desirable. As the polymer compound, polyvinylpyrrolidone, Eudragit (such as methacrylic acid-methyl methacrylate copolymer L, methacrylic acid-methyl methacrylate copolymer S, aminoalkyl methacrylate copolymer E, aminoalkyl methacrylate copolymer S, methacrylic acid-ethyl acrylate copolymer RL, and methacrylic acid-ethyl acrylate copolymer RS), crospovidone, and carboxyvinyl polymers are desirable. As the organic acid, acetic acid, lactic acid, and citric acid are desirable. One of these dispersion aids can be used alone, or two or more thereof can be used in combination.

When including the dispersion aid, the blending amount thereof is preferably 0.5 to 50 mass%, more preferably 1 to 20 mass%, and particularly preferably 1 to 10 mass% based on the total mass of the adhesive composition.

As the antioxidant, tocopherol and ester derivatives thereof, ascorbic acid, ascorbyl stearate, nordihydroguaiaretic acid, dibutylhydroxytoluene (BHT), butylated hydroxyanisole, sodium pyrosulfite, and sodium sulfite are desirable, and particularly for use as a solubilizer in the production process, use of dibutylhydroxytoluene (BHT) is particularly preferable.

As the filler, aluminum hydroxide, calcium carbonate, magnesium carbonate, silicates (such as aluminum silicate and magnesium silicate), silicic acid, barium sulfate, calcium sulfate, calcium zincate, zinc oxide, and titanium oxide are desirable.

As the preservative, disodium edetate, tetrasodium edetate, ethyl parahydroxybenzoate, propyl parahydroxybenzoate, and butyl parahydroxybenzoate, are desirable.

As the ultraviolet ray absorber, p-aminobenzoic acid derivatives, anthranilic acid derivatives, salicylic acid derivatives, coumarin derivatives, amino acid compounds, imidazoline derivatives, pyrimidine derivatives, and dioxane derivatives are desirable.

The antioxidants, fillers, preservatives, and ultraviolet ray absorbers as described above are preferably contained in a total amount of 10 mass% or less based on the total mass of the adhesive composition.

Although the production method of the adhesive patch 10 is not particularly limited, for example, the adhesive patch 10 can be produced by melting adhesive compositions containing drugs (ropinirole compounds), adhesives, and softeners, followed by mixing to homogeneity, and applying the resulting mixture to a release liner to form an adhesive layer, and then sticking the resulting adhesive layer to a backing.

The adhesive patch 10 can also be produced by dissolving the adhesive composition into a solvent such as toluene, hexane, heptane, and ethyl acetate, and spreading the resulting mixture over a release liner, followed by drying to remove the solvent to form an adhesive layer, and then sticking the resulting adhesive layer to a backing.

As described above, in the adhesive patch-containing package bag 1 according to the present embodiment, the packaged oxygen scavenger 20 as deoxygenation means is stored in the package bag 8 together with the adhesive patch 10. This packaged oxygen scavenger 20 is constituted by an oxygen scavenger package bag 24 and an oxygen scavenger 22 stored in this package bag 24.

As the packaged oxygen scavenger 20, for example, Ageless (Mitsubishi Gas Chemical Company, Inc.), StabilOx (Multisorb Technologies), Well Pack (Taisei Co., Ltd.), Ever Fresh (Torishige Sangyo Co., Ltd.), Oxy-eater (Ueno Fine Chemicals Industry, Ltd.), Keepit (Dorency Co., Ltd.), Keplon (Keplon Co., Ltd.), Sanso-cut (Iris Fine Products Co., Ltd.), Sansoresu (Hakuyo, Inc.), Sequl (Nisso Jushi Co., Ltd.), Tamotsu (OhE Chemicals Inc.), Vitalon (Tokiwa Sangyo), Modulan (Nippon Kayaku Food Techno Co., Ltd.), Wonder Keep (Powdertech Co., Ltd.), and Freshness-keeping agent C (Toppan Printing Co., Ltd.) can be preferably used directly or in an appropriately packaged form.

Also, examples of deoxygenation means not involving the packaged oxygen scavenger 20 include a method of mixing powder having deoxygenation actions such as aluminum, zinc, manganese, copper, iron, hydrosulfite, and activated carbon into a layer constituting the package bag, such as the sealant layer.

The relative humidity in the space inside the package bag according to the present embodiment is maintained at 35% or higher, preferably 41% or higher, and more preferably 41% to 70%. Ropinirole compounds can be more stably stored by intentionally setting the humidity higher than that conventionally used. That is, when the humidity in the package bag is set less than 35%, crystals of the ropinirole compound in the adhesive patch precipitate; therefore, it is not preferable.

The relative humidity in the space inside the package bag can be adjusted so as to be within the aforementioned range by a method of adjusting the manufacturing environment to certain temperature and humidity during the production process and a method of adjusting the humidity by the moisture in the oxygen scavenger.

### Examples

Hereinbelow, the present invention is described in detail with reference to Examples and Comparative Examples.

### [Production Example of an adhesive patch and a package bag]

### (Example 1)

### (Production of package bag A)

Two multilayer films A in which low density polyethylene (LDPE), an aluminum foil (Al), polyethylene (PE), and cellophane (PT) were laminated in this order were prepared. Subsequently, these two multilayer films A were placed so that the layers of LDPE faced each other, and the films were bonded at the peripheral three sides by thermal fusion, whereby a square package bag A with one side open for storing an adhesive patch therefrom was obtained.

### (Production of adhesive patch B)

Using a mixer, ropinirole hydrochloride, sodium hydroxide, liquid paraffin, and methanol (solvent) were mixed in accordance with the blending ratio as shown in Table 1, followed by adding and mixing a mixed solution of SIS, cycloaliphatic hydrocarbon resin, and toluene to give an adhesive solution. The resulting adhesive solution was spread over a film, followed by drying to remove the solvent to form an adhesive layer. On this adhesive layer, a polyethylene terephthalate film was laminated as a backing, whereby an adhesive patch B was obtained. The adhesive patch B thus obtained was cut into 6.25-square-cm pieces (square). It is to be noted that the thickness of the adhesive layer of the adhesive patch B was 100 µm.

### (Enclosure of the adhesive patch into the package bag)

The adhesive patch B and a deoxygenation pack containing an iron-based oxygen scavenger (the product of Mitsubishi Gas Chemical Company, Inc., deoxygenation ability: 15 cm³, size: 7.3 cm x 5 cm) were enclosed in the package bag A under the environment of 23°C and a relative humidity of 38%, and after storing the resulting package bag A at 4°C for 30 days, the humidity inside the bag was measured.

**[Table 1]**

| | Blending ratio (mass%) |
|---|---|
| Ropinirole hydrochloride | 5.0 |
| Sodium hydroxide | 0.5 |
| Liquid paraffin | 21.6 |
| SIS | 27.0 |
| Cycloaliphatic saturated hydrocarbon resin | 45.9 |

Adhesive patch-containing package bags were produced by the method described below in accordance with Table 2 in Examples 2 to 3 and Comparative Examples 1 to 4.

**[Table 2]**

| | Multilayer film | Enclosure |
|---|---|---|
| Example 1 | LDPE/Al/PE/Cellophane | Deoxygenation pack containing iron-based oxygen scavenger (Deoxygenation ability 15cm³, Size 7.3cm×5cm) |
| Example 2 | LDPE/Al/PE/Cellophane | Deoxygenation pack containing iron-based oxygen scavenger (Deoxygenation ability 15cm³, Size 11cm×7.5cm) |
| Example 3 | PAN/Al/PE/Cellophane | Deoxygenation pack containing iron-based oxygen scavenger (Deoxygenation ability 15cm³, Size 7.3cm×5cm) |
| Comparative Example 1 | LDPE/Al/PE/Cellophane | None |
| Comparative Example 2 | LDPE/Al/PE/Cellophane | Deoxygenation pack with a drying function |
| Comparative Example 3 | LDPE/Al/PE/Cellophane | Commercial desiccant |
| Comparative Example 4 | LDPE/Al/PE/Cellophane | Nitrogen replacement |

### (Example 2)

The adhesive patch B and a deoxygenation pack containing an iron-based oxygen scavenger (the product of Mitsubishi Gas Chemical Company, Inc., deoxygenation ability: 15 cm³, size: 11 cm × 7.5 cm) were enclosed in the package bag A under the environment of 23°C and a relative humidity of 38%, and in a similar manner to Example 1, the resulting package bag A was stored at 4°C for 30 days, and the humidity inside the bag was measured.

### (Example 3)

### (Production of package bag B)

Two multilayer films A in which polyacrylonitrile (PAN), an aluminum foil (Al), polyethylene (PE), and cellophane (PT) were laminated in this order were prepared. Subsequently, these two multilayer films A were placed so that the layers composed of PAN face each other, and in a similar manner to Example 1, a package bag B was obtained.

### (Enclosure of the adhesive patch into the package bag)

The adhesive patch B and a deoxygenation pack containing an iron-based oxygen scavenger (the product of Mitsubishi Gas Chemical Company, Inc., deoxygenation ability: 15 cm³, size: 7.3 cm × 5 cm) were enclosed in the package bag B under the environment of 23 °C and a relative humidity of 38%, and after storing the resulting package bag B at 4°C for 30 days, the humidity inside the bag was measured.

### (Comparative Example 1)

### (Enclosure of the adhesive patch into the package bag)

The adhesive patch B was enclosed in the package bag A under the environment of 23°C and a relative humidity of 38%, and after storing the resulting package bag A at 4°C for 30 days, the humidity inside the bag was measured.

### (Comparative Example 2)

### (Enclosure of the adhesive patch into the package bag)

The adhesive patch B and a deoxygenation pack with a drying function (trade name: PharmaKeep KC-20, the product of Mitsubishi Gas Chemical Company, Inc.) were enclosed in the package bag A under the environment of 23°C and a relative humidity of 38%, and after storing the resulting package bag A at 4°C for 30 days, the humidity inside the bag was measured.

### (Comparative Example 3)

### (Enclosure of the adhesive patch into the package bag)

The adhesive patch B and a commercial desiccant pack (Sud Chemie) were enclosed in the package bag A under the environment of 23°C and a relative humidity of 38%, and after storing the resulting package bag A at 4°C for 30 days, the humidity inside the bag was measured.

### (Comparative Example 4)

### (Enclosure of the adhesive patch into the package bag)

The adhesive patch B was enclosed in the package bag A with nitrogen replacement, and after storing the resulting package bag A at 4°C for approximately seven days, the humidity inside the bag was measured.

### (Humidity measurement method)

The humidity inside the adhesive patch-containing package bag produced in each of Examples and Comparative Examples was measured by enclosing a small humidity measurement device (DICKSON TK500) into the package bag together with the adhesive patch. It should be noted that this device can measure the relative humidity in the measurement environment and electronically record the measurement data, and upon completion of the measurement, the recorded humidity data can be read and analyzed.

### (Results of measurement of humidity)

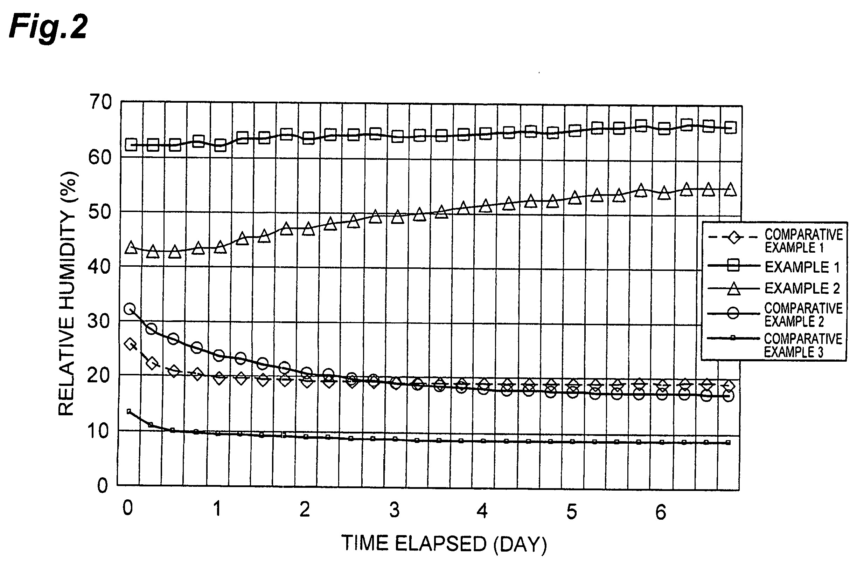

As a result of the measurement of the relative humidity inside the adhesive patch-containing package bag produced in each of Examples 1 and 2 and Comparative Examples 1 to 3, as shown in Figure 2, while the relative humidity inside the adhesive patch-containing package bags of Examples 1 and 2 was maintained at 41 % or higher, the relative humidity inside the adhesive patch-containing package bags of Comparative Examples 1 to 3 shifted below a relative humidity of 41%.

### (Evaluation on storage stability of the adhesive patch)

The coloration with time, precipitation of crystals, and production of decomposition products were evaluated in the adhesive patch-containing package bag produced in each of Examples and Comparative Examples by the method below.

### (Evaluation method of coloration of adhesive patch with time)

After storing the adhesive patch-containing package bags of Examples and Comparative Examples under the storage conditions as shown in Table 3, the adhesive patch was removed from the package bag, and coloration of the adhesive layer was visually evaluated in accordance with the following criteria. The results thus obtained are shown in Table 3.
A: No change in color from the beginning
B: Slightly colored but no problem for practical use
C: Obviously colored and there is a problem for practical use

**[Table 3]**

| (Evaluation results of coloration of the adhesive patch with time) | |
|---|---|
| Sample (Storage condition) | Evaluation |
| Example 1 (Initial period) | A |
| Example 1 (60°C-1M) | A |
| Example 3 (60°C-1M) | B |
| Comparative Example 4 (60°C-1M) | C |
| Example 1 (40°C-1M) | A |
| Comparative Example 1 (40°C-1M) | C |

| | |
|---|---|
| 1M: 1 month | |

No coloration was observed in the adhesive patch of Example 1, whereas slight coloration was observed in the adhesive patch of Example 3 after storage at 60°C. Obvious coloration was observed in the adhesive patches of Comparative Examples 1 and 4.

### (Evaluation method of the precipitation of drug crystals in the adhesive patch)

The adhesive patch-containing package bags of Examples and Comparative Examples were stored at 4°C for 30 days, in which the humidity was measured by the aforementioned method, and when radial needle crystals were found to be precipitated in a part of or the entire adhesive patch, the precipitation of crystals was evaluated as "yes", and when there was no change, the precipitation of crystals was evaluated as "no", by visual or microscopic observations. The results thus obtained are shown in Table 4.

**[Table 4]**

| (Results of evaluation of the precipitation of drug crystals in the adhesive patch) | | |
|---|---|---|
| Sample (Storage condition) | Humidity (Relative humidity %) | Precipitation of crystals |
| Example 1 (4°C-30 days) | 41 or higher | No |
| Example 2 (4°C-30 days) | 41 or higher | No |
| Example 3 (4°C-30 days) | 41 or higher | No |
| Comparative Example 1 (4°C-30 days) | 30 or lower | Yes |
| Comparative Example 2 (4°C-30 days) | 30 or lower | Yes |
| Comparative Example 3 (4°C-30 days) | 30 or lower | Yes |
| Comparative Example 4 (4°C-30 days) | n.d. | Yes |

| | | |
|---|---|---|
| n.d.: not detected | | |

### (Evaluation method of drugs and the production of decomposition products)

After storing the adhesive patch-containing package bags of Examples and Comparative Examples under the storage conditions as shown in Table 5, the adhesive patches were removed from the package bags. The components contained in the adhesive patches were extracted with 10 mL of tetrahydrofuran and 40 mL of a water/methanol mixed solution to give sample solutions, and ropinirole and each unidentified compound were searched by high-performance liquid chromatography (ODS column, UV detector).

The amount of each unidentified compound was calculated from the absorption area of the chromatography of each compound by setting the theoretical amount of ropinirole at 100. It should be noted that when no unidentified compound was detected, the result was expressed as "-."

**[Table 5]**

| (Results of quantitation of drugs and unidentified products) | | |
|---|---|---|
| Sample (Storage condition) | % Drug content | % Unidentified compound relative to drug/retention time |
| | | 26.9 min. |
| Example 1 (Initial period) | 100.0 | - |
| Example 1 (60°C-1M) | 99.5 | 0.24 |
| Example 3 (60°C-1M) | 102.8 | 0.08 |
| Comparative Example 4 (60°C-1M) | 94.6 | 1.91 |
| Example 1 (40°C-1M) | 99.8 | 0.08 |
| Comparative Example 1 (40°C-1M) | 80.4 | 2.28 |

As a result, as shown in Table 5, the main decomposition products were observed in the adhesive patches of Examples and Comparative Examples at a retention time of 26.9 minutes; however, compared to Comparative Examples, the amount of decomposition products in Examples was extremely small and the content of ropinirole was maintained at a high level.

### Industrial Applicability

The adhesive patch-containing package bag and the method for storing an adhesive patch according to the present invention can inhibit the precipitation of crystals and provide a medical adhesive patch having excellent storage stability of the drugs, and thus, are industrially useful.

### Reference Signs List

1...Adhesive patch-containing package bag, 2...Sealant layer, 4...Barrier layer, 6...Outer layer, 7, 7a, 7b...Laminated packing material, 8...Package bag, 10...Adhesive patch, 12...Backing, 14...Adhesive layer, 16...Release liner, 20...Packaged oxygen scavenger, 22...Oxygen scavenger, 24... Oxygen scavenger package bag

## Claims

1. An adhesive patch-containing package bag comprising:
a package bag; and
an adhesive patch stored inside the package bag,
wherein the package bag comprises a sealant layer as an innermost layer, an outer layer as an outmost layer and a barrier layer made of aluminum between the sealant layer and the outer layer,
the package bag further comprises an oxygen scavenger as deoxygenation means,
the adhesive patch comprises a backing, an adhesive layer laminated on at least one side of the backing and containing ropinirole and/or a pharmaceutically acceptable salt thereof, and a release liner covering the adhesive layer, and
a relative humidity at 4°C is maintained at 35% or higher inside the package bag,
wherein the sealant layer is made of a resin selected from the group consisting of polyacrylonitrile, polyethylene and polyester.

2. The adhesive patch-containing package bag according to claim 1, wherein the outer layer is made of a resin selected from the group consisting of polyethylene terephthalate, cellophane and biaxially-drawn polypropylene.

3. The adhesive patch-containing package bag according to claim 1 or 2, wherein the adhesive layer comprises at least one adhesive selected from the group consisting of an acrylic adhesive, a rubber adhesive and a silicone adhesive.

4. A method for storing an adhesive patch, comprising:
storing an adhesive patch into a package bag, the adhesive patch comprising a backing, an adhesive layer laminated on at least one side of the backing and containing ropinirole and/or a pharmaceutically acceptable salt thereof, and a release liner covering the adhesive layer; and
maintaining a relative humidity at 4°C inside the package bag at 35% or higher,
wherein the package bag comprises a sealant layer as an innermost layer, an outer layer as an outmost layer and a barrier layer made of aluminum between the sealant layer and the outer layer and the package bag further comprise an oxygen scavenger as deoxygenation means, and
wherein the sealant layer is made of a resin selected from the group consisting of polyacrylonitrile, polyethylene and polyester.

## Patentansprüche

1. Heftpflaster enthaltender Packungsbeutel, umfassend:
einen Packungsbeutel; und
ein im Inneren des Packungsbeutels aufbewahrtes Heftpflaster,
wobei der Packungsbeutel eine Dichtungsmaterialschicht als eine innerste Schicht, eine Außenschicht als eine äußerste Schicht und eine aus Aluminium gefertigte Sperrschicht zwischen der Dichtungsmaterialschicht und der Außenschicht umfasst,
der Packungsbeutel außerdem einen Sauerstofffänger als Sauerstoffentzugsmittel umfasst,
das Heftpflaster einen Träger, eine Klebstoffschicht, die auf wenigstens eine Seite des Trägers laminiert ist und Ropinirol und/oder ein pharmazeutisch verträgliches Salz davon enthält, und eine Trennlage, die die Klebstoffschicht bedeckt, umfasst, und
eine relative Feuchtigkeit bei 4 °C bei 35 % oder höher im Inneren des Packungsbeutels gehalten wird,
wobei die Dichtungsmaterialschicht aus einem Harz gefertigt ist, das aus der Gruppe bestehend aus Polyacrylnitril, Polyethylen und Polyester ausgewählt ist.

2. Heftpflaster enthaltender Packungsbeutel gemäß Anspruch 1, wobei die Außenschicht aus einem Harz gefertigt ist, das aus der Gruppe bestehend aus Polyethylenterephthalat, Cellophan und biaxial gerecktem Polypropylen ausgewählt ist.

3. Heftpflaster enthaltender Packungsbeutel gemäß Anspruch 1 oder 2, wobei die Klebstoffschicht wenigstens einen Klebstoff umfasst, der aus der Gruppe bestehend aus einem Acrylklebstoff, einem Kautschukklebstoff und einem Siliconklebstoff ausgewählt ist.

4. Verfahren zum Aufbewahren eines Heftpflasters, umfassend:
das Aufbewahren eines Heftpflasters in einem Packungsbeutel, wobei das Heftpflaster einen Träger, eine Klebstoffschicht, die auf wenigstens eine Seite des Trägers laminiert ist und Ropinirol und/oder ein pharmazeutisch verträgliches Salz davon enthält, und eine Trennlage, die die Klebstoffschicht bedeckt, umfasst; und
das Halten einer relativen Feuchtigkeit bei 4 °C im Inneren des Packungsbeutels bei 35 % oder höher,
wobei der Packungsbeutel eine Dichtungsmaterialschicht als eine innerste Schicht, eine Außenschicht als eine äußerste Schicht und eine aus Aluminium gefertigte Sperrschicht zwischen der Dichtungsmaterialschicht und der Außenschicht umfasst und der Packungsbeutel außerdem einen Sauerstofffänger als Sauerstoffentzugsmittel umfasst, und
wobei die Dichtungsmaterialschicht aus einem Harz gefertigt ist, das aus der Gruppe bestehend aus Polyacrylnitril, Polyethylen und Polyester ausgewählt ist.

## Revendications

1. Sac de conditionnement contenant un timbre adhésif comprenant :
un sac de conditionnement ; et
un timbre adhésif stocké à l'intérieur du sac de conditionnement,
où le sac de conditionnement comprend une couche de scellement comme couche la plus interne, une couche externe comme couche la plus externe et une couche formant barrière constituée d'aluminium entre la couche de scellement et la couche externe,
le sac de conditionnement comprend en outre un épurateur d'oxygène comme moyen de désoxygénation,
le timbre adhésif comprend un renfort dorsal, une couche adhésive stratifiée sur au moins une face du renfort dorsal et contenant du ropinirole et/ou son sel pharmaceutiquement acceptable, et une protection antiadhésive recouvrant la couche adhésive, et
une humidité relative à 4°C est maintenue à 35 % ou plus à l'intérieur du sac de conditionnement,
où la couche de scellement est constituée d'une résine sélectionnée dans le groupe constitué du polyacrylonitrile, du polyéthylène et du polyester.

2. Sac de conditionnement contenant un timbre adhésif selon la revendication 1, où la couche externe est constituée d'une résine sélectionnée dans le groupe constitué du poly(téréphtalate d'éthylène), du cellophane et du polypropylène étiré de manière biaxiale.

3. Sac de conditionnement contenant un timbre adhésif selon la revendication 1 ou 2, où la couche adhésive comprend au moins un adhésif sélectionné dans le groupe constitué d'un adhésif acrylique, d'un adhésif à base de caoutchouc et d'un adhésif de silicone.

4. Procédé de stockage d'un timbre adhésif, comprenant :
le stockage d'un timbre adhésif dans un sac de conditionnement, le timbre adhésif comprenant un renfort dorsal, une couche adhésive stratifiée sur au moins une face du renfort dorsal et contenant du ropinirole et/ou son sel pharmaceutiquement acceptable, et une protection antiadhésive recouvrant la couche adhésive ; et
le maintien d'une humidité relative à 4°C à l'intérieur du sac de conditionnement à 35 % ou plus,
où le sac de conditionnement comprend une couche de scellement comme couche la plus interne, une couche externe comme couche la plus externe et une couche formant barrière constituée d'aluminium entre la couche de scellement et la couche externe et le sac de conditionnement comprend en outre un épurateur d'oxygène comme moyen de désoxygénation, et
où la couche de scellement est constituée d'une résine sélectionnée dans le groupe constitué du polyacrylonitrile, du polyéthylène et du polyester.
